# FASCICULE DE BREVET EUROPEEN

(11) **EP 1 282 817 B1**
(45) Date de publication et mention de la délivrance du brevet: **27.04.2005**
(21) Numéro de dépôt: 01936518.8
(22) Date de dépôt: 15.05.2001
(51) Int. Cl.: G01N 33/533, G01N 33/86, G01N 15/10

(54) **MONOREACTIF POUR LE DOSAGE DES MICROPARTICULES PLAQUETTAIRES**
REAGENZ ZUR BESTIMMUNG VON BLUTPLÄTTCHEN-MIKROTEILCHEN
MONOREAGENT FOR ASSAYING PLATELET-DERIVED MICROPARTICLES

(30) Priorité: 16.05.2000 FR 0006247
(43) Date de publication de la demande: 12.02.2003
(73) Titulaire: Biocytex, 13010 Marseille (FR)
(72) Inventeur: CANTON, Michel, F-92300 Levallois-Perret (FR); BOTOSEZZY, Isabelle, F-13013 Marseille (FR)
(74) Mandataire: Warcoin, Jacques
(86) Numéro de dépôt international: PCT/FR2001/001468
(87) Numéro de publication internationale: WO 2001/088539

(56) Documents cités:
- WO-A-91/00509
- FR-A- 2 795 820
- US-A- 5 380 663
- COMBES V ET AL: "IN VITRO GENERATION OF ENDOTHELIAL MICROPARTICLES AND POSSIBLE PROTHROMBOTIC ACTIVITY IN PATIENTS WITH LUPUS ANTICOAGULANT" JOURNAL OF CLINICAL INVESTIGATION,US,NEW YORK, NY, vol. 104, no. 1, juillet 1999 (1999-07), pages 93-102, XP000913435 ISSN: 0021-9738
- SCHARF RUEDIGER E ET AL: "Activation of platelets in blood perfusing angioplasty-damaged coronary arteries: Flow cytometric detection." ARTERIOSCLEROSIS AND THROMBOSIS, vol. 12, no. 12, 1992, pages 1475-1487, XP000998111 ISSN: 1049-8834
- LATGER V ET AL: "PATHOLOGIE VASCULAIRE ET ACTIVATION CELLULAIRE. EXPLORATION DU PH-NOTYPE CELLULAIRE ADH-RENT PAR CYTOM-TRIE EN FLUX QUANTITATIVE" JOURNAL DES MALADIES VASCULAIRES,FR,MASSON, PARIS, vol. 24, no. 1, 1999, pages 11-18, XP000910966 ISSN: 0398-0499
- ABRAMS C S ET AL: "DIRECT DETECTION OF ACTIVATED PLATELETS AND PLATELET-DERIVED MICROPARTICLES IN HUMANS" BLOOD,US,W.B. SAUNDERS, PHILADELPHIA, VA, vol. 75, no. 1, 1990, pages 128-138, XP000564281 ISSN: 0006-4971
- MATZDORFF A C ET AL: "Relative and absolute changes of activated platelets, microparticles and platelet aggregates after activation in vitro." HAEMOSTASIS, vol. 28, no. 6, novembre 1998 (1998-11), pages 277-288, XP001023452 ISSN: 0301-0147

## Description

La présente invention concerne un monoréactif et un kit de diagnostic le comprenant, pour la détection et la quantification des microparticules d'origine plaquettaire (MPP) par marquage en double couleur.

Les plaquettes activées sont connues pour relarguer des vésicules appelées microparticules plaquettaires (MPP). Ces particules de faible diamètre (pouvant cependant aller de 0,1 à 0,8 µm) résultent d'une vésiculation de la membrane plasmatique plaquettaire après certains stimuli (thrombine, collagène, C5b9...). De par leur activité prothrombotique et procoagulante, les MPP semblent jouer un rôle important in vivo.
Exprimant plusieurs récepteurs plaquettaires (Gplb, GpIIb/IIIa..,), les MPP sont capables de fixer plusieurs types de ligands plaquettaires (fibrinogène, fibronectine, collagène, vWF, vitronectine...) et d'adhérer à la matrice sub-endothéliale, au niveau d'une brèche vasculaire. Des taux élevés de MPP circulantes ont été observés dans de nombreuses pathologies telles que : angor instable, infarctus du myocarde, angiographie coronarienne, diabète sucré, dérivation cardiopulmonaire, hémoglobinurie paroxystique nocturne, anémie aplasique, HIT (thrombopénies induites par l'héparine), purpura thrombopénique idiomathique ... Inversement, certaines pathologies comme le syndrome de Scott peuvent être associées à une déficience dans le taux de MPP circulantes.
Ainsi, la détection et la numération de ces MPP semble être un critère important dans la détection, et l'évaluation de la sévérité d'un état prothrombotique au cours de différentes pathologies.

Les phénomènes liés à la formation des microparticules d'origine plaquettaire et l'intérêt de leur détection pour le diagnostic ont été largement décrits dans la littérature (1-5).

Différentes approches basées sur des techniques telles que par exemple la filtration (6), l'ELISA (7), ou plus particulièrement la cytométrie de flux (8-11) ont ainsi été proposées pour leur isolement et/ou leur quantification.

Combes, V. et al, J. Clin. Invest., 1999, Vol. 104, pages 93-102 divulgue un monoréactif pour la détection et quantification des MPP pour un double marquage des MPP.

Le principe de la cytométrie de flux et ses avantages pour discriminer et quantifier des sous-populations de cellules et particules cellulaires sont aujourd'hui bien connus de l'homme du métier.
Cette méthode a déjà été utilisée pour la détection des plaquettes activées dans des échantillons sanguins et la détermination de microparticules d'origine plaquettaire procoagulantes par des tests fonctionnels (12).
Toutefois, les études réalisées utilisent pour la plupart un marquage fluorescent en une seule couleur, et/ou mettent en oeuvre des billes de comptage dont les performances en terme de calibration sont insuffisantes pour concevoir une méthode industrialisable permettant de réaliser des tests de détection et quantification des MPP d'une manière totalement standardisée,

La présente invention apporte un perfectionnement aux méthodes préexistantes en proposant une nouvelle solution qui permet en un temps, et de manière standardisée, de détecter et de compter spécifiquement des microparticules d'origine plaquettaire dans un échantillon sanguin, sans traitement préalable.
A cet effet, un monoréactif particulier, associant plusieurs populations de billes calibrées et un double marquage des MPP, a été mis a point. Ainsi, un tel réactif permet à la fois 1) un isolement optimal des MPP sur un critère de taille, 2) leur caractérisation par des marquages spécifiques, et 3) leur numération au moyen de billes de comptage. Il offre donc un moyen de détecter spécifiquement et de compter, de façon très fiable, le nombre de MPP dans un échantillon, même lorsque celles-ci sont présentes en très faible quantité.

La présente invention a ainsi pour objet un monoréactif pour la détection et la quantification des microparticules plaquettaires (MPP) dans un échantillon sanguin par cytométrie de flux, comprenant ;
a) un réactif 1 pour un double marquage des MPP comprenant :
   - soit de l'Annexine V ou tout autre marqueur spécifique des phospholipides membranaires couplé à un fluorochrome 1,
   - soit une population 1 d'anticorps monoclonaux (ACM) dirigés contre des structures membranaires plaquettaires marqués avec un fluorochrome 1, **(réactif 1a)**, et,
   une population 2 d'ACM dirigés contre des structures membranaires plaquettaires marqués avec un fluorochrome 2 **(réactif 1b)**, ledit fluorochrome 2 étant différent du fluorochrome 1 et,
b) **un réactif 2** constitué par un mélange de microsphères comprenant :
   - une population de microsphères A marquée par l'un des deux fluorochromes 1 ou 2 ou autre fluorochrome de spectre similaire à l'un des deux, servant à définir la région d'analyse des MPP en terme de taille (paramètres de diffusion lumineuse) et permettant leur différenciation (billes de positionnement),
   - une population de microsphères B non marquée ou marquée par l'un des deux fluorochromes 1 ou 2 ou autre fluorochrome de spectre similaire à l'un des deux, dont la concentration est connue, servant d'étalon interne au comptage des microparticules (billes de comptage),
   - une population de microsphères C marquée uniquement par le fluorochrome 1, servant à définir sur le paramètre de fluorescence du fluorochrome 1 le seuil d'intensité minimum qui délimite la région d'analyse des microparticules sur lesquelles sont fixés les ACM 1 ou l'annexine V ou tout autre marqueur spécifique des phospholipides membranaires marqués au fluorochrome 1 (billes seuils),
   - et une population de microsphères D, de diamètre identique à C, et marquée uniquement par le fluorochrome 2, servant à définir sur le paramètre de fluorescence du fluorochrome 2 le seuil d'intensité minimum qui délimite la région d'analyse des microparticules sur lesquelles sont fixés les ACM 2 marqués au fluorochrome 2 (billes seuils).

Le monoréactif selon l'invention possède donc plusieurs fonctions, qui sont :
- marquage des microparticules en double couleur, permettant une différenciation optimale des microparticules plaquettaires des équivalents d'origine autre,
- standardisation des seuils de discrimination des microparticules d'intérêt des autres particules et débris responsables d'un bruit de fond éventuel,
- standardisation du comptage des microparticules plaquettaires marquées et discriminées par le comptage de référence de la bille étalon interne.

Avantageusement, chaque population C et D de billes seuils est composée de deux sous-populations de billes de 2 niveaux de fluorescence 1 ou 2 pour optimiser la standardisation de l'analyse par un positionnement des seuils et un réglage des compensations.

Les différentes populations de billes peuvent être obtenues à partir de divers matériaux classiquement utilisés pour la fabrication de ce type de particules. Ce sont par exemple les polymères organiques, tels que les polysaccharides, les polymères de styrène, les polyacrylates, la polyacrylamide, l'hydroxyéthyl polyméthacrylate, les polyvinyles, les polystyrènes et les polymères contenant des groupes aromatiques. Un matériau préférentiellement utilisé est le polystyrène. Le matériau peut toutefois être différent entre les différentes populations de billes utilisées.

Plusieurs fluorochromes sont disponibles dans le commerce. On utilise avantageusement la phycoerythrine (PE) ou la fluoresceine, comme par exemple l'isothiocyanate de fluoresceine (FITC).

Les billes de comptage, dont le rôle est de numérer les MPP, ont avantageusement un diamètre supérieur ou égal à 5 µm, pour qu'en cytométrie le nuage de billes de comptage soit bien distinct des cellules et des billes d'une autre population (billes seuils). Préférentiellement, leur diamètre est compris entre 5 et 10 µm.

Ces billes de comptage peuvent être non marquées ou marquées, dans la masse ou en surface. Elles sont préférentiellement marquées dans la masse par l'un des fluorochromes 1 ou 2 utilisés pour le marquage des billes seuils, ou autre fluorochrome de spectre similaire à l'un d'eux.

Les billes de positionnement permettent de positionner la fenêtre d'analyse des MPP selon la taille. Leur diamètre est avantageusement compris entre 1 et 0,5 µm. Elles sont marquées par l'un des deux fluorochromes 1 et 2 ou autre fluorochrome de spectre similaire à l'un des deux dans la masse, ou en surface.

Les billes seuils ont pour rôle de standardiser l'analyse en positionnant la fenêtre d'analyse des MPP et permettre le réglage des compensations. Leur diamètre est compris entre 1 et 5 µm et est préférentiellement de 3 µm.

Les deux populations C et D de billes seuils consistent en deux populations marquées par l'un des fluorochromes 1 ou 2 et sont avantageusement constituées toutes deux de deux catégories de billes de même nature mais présentant des niveaux de fluorescence différents.
Ces calibrants sont avantageusement étalonnés en MESF (Molécules of Equivalent Soluble Fluorochrome - 13) grâce à un étalon (Quantum 24 fluorescein - Code : RF 824 ; Quantum PE R-Phycoerythrin - Code RF 827, FCSC Europe).

L'intensité des billes seuils est ainsi réglée pour être à la limite entre l'intensité minimale des MPP et l'intensité maximale des microparticules d'origine autre et des contaminants de taille similaire (poussières, débris cellulaires ...).
La valeur des seuils fluorochrome 1 et fluorochrome 2, préférentiellement FITC et PE, est déterminée sur une cohorte d'individus normaux et pathologiques grâce aux calibrants étalons commerciaux, pour une discrimination optimale des MPP par rapport aux autres contaminants.
La valeur des seuils ainsi définis donne la valeur des billes seuils associés (C, D) du monoréactif.

Les anticorps monoclonaux (ACM) des populations 1 et 2 sont avantageusement dirigés contre des structures membranaires plaquettaires choisies parmi les spécificités suivantes : CD61, CD41, CD42a, CD42b, CD42c, CD49b, CD29, CD62P, CD63, protéine S et prothrombine.
Ces populations 1 et 2 peuvent être représentées par un seul ACM, par plusieurs ACM de même spécificité mais dirigés contre des épitopes différents, ou par plusieurs ACM de spécificité plaquettaire différente.

Selon une variante préférée, le réactif 1 du monoréactif de l'invention est constitué d'annexine V ou autre marqueur spécifique des phospholipides membranaires marqué par un fluorochrome 1 (réactif 1a), et d'une population 2 d'ACM marqués par un fluorochrome 2 (réactif 1b).
On utilise de préférence une population 2 d'ACM comprenant plusieurs ACM de spécificités différentes, et plus préférentiellement des ACM anti-CD61, et des ACM anti-CD42b. Un exemple de réactif préféré est constitué par des ACM anti-CD61 et anti-CD42b marqués à la PE, et par de l'Annexine V marquée au FITC.

Selon une variante avantageuse, les anticorps anti-CD61 sont produits par les hybridomes P18 et 4F8, déposés auprès de la BCCM/LMBP Collection (Belgium Coordinated Collections of Microorganisms) selon le Traité de Budapest respectivement les 26.06.97 et 02.06.98 sous les N° LMBP162CB (P18) et LMBP1667CB (4F8).
L'association de ces deux ACM est particulièrement avantageuse car il n'y a pas de gène stérique entre eux, et leur fixation sur les MPP n'est pas altérée par la présence éventuelle d'agents anti-agrégants anti-GPIIb/IIIa commerciaux tels que le Réopro (Abciximab, Centocor), l'Integrilin (Ceptifibatide, Shering-Plough), l'Agrastat (Tirofiban, Merck).

Les ACM anti-CD42b sont par exemple représentés par l'anticorps SZ2. Celui-ci peut être obtenu auprès de la société Beckman Coulter/Immunotech (Réf. IM0409).

L'Annexine V est disponible commercialement. Elle peut par exemple provenir de chez Bender (Réf. BMS306 FI).
D'autres composés utilisables comme marqueurs spécifiques des phospholipides membranaires sont par exemple des anticorps anti-phosphatidylserine, tels les anticorps BA3B5C4 et 3SB9b décrits dans la littérature (14) ou les anticorps Ab-1 distribués par France Biochem (Réf. AM31, Oncogene Research Product).
Avantageusement, l'échantillon est constitué par du sang total prélevé sur CTAD (citrate théophilline adenosine dipyridamole), citrate de sodium ou EDTA. Il peut aussi être constitué par du plasma, bien que ce type d'échantillon, du fait de l'étape de préparation préalable qu'il nécessite, ne soit pas utilisé préférentiellement.

Selon un deuxième aspect, l'invention a trait à un kit de diagnostic comprenant un monoréactif tel que mentionné ci-dessus, destiné à la détection et à la quantification des MPP dans un échantillon sanguin.

Un tel kit de diagnostic comprend ainsi avantageusement :
- **un réactif 1a** constitué par une population 1 d'ACM spécifiques de structures membranaires plaquettaires, ou par de l'Annexine V ou tout autre marqueur spécifique des phospholipides membranaires, marqués avec un fluorochrome 1,
- **un réactif 1b** constitué par une population 2 d'ACM spécifiques de structures membranaires plaquettaires, marqués avec un fluorochrome 2, ledit fluorochrome 2 étant différent du fluorochrome 1,
- **un réactif 2** constitué par :
   - une population de billes de positionnement de la fenêtre d'analyse des MPP marquée par le fluorochrome 1 ou 2, ou autre fluorochrome de spectre similaire à l'un des deux,
   - une population de billes de comptage non marquée ou marquée par le fluorochrome 1 ou 2, ou autre fluorochrome de spectre similaire à l'un des deux,
   - une population de billes seuils, préférentiellement constituée par deux sous-populations de billes de deux niveaux, marquées par le fluorochrome 1,
   - une population de billes seuils, préférentiellement constituée par deux sous-populations de billes de deux niveaux marquées par le fluorochrome 2, et, de préférence,
- **un réactif 3** constitué par un tampon de dilution.
   Préférentiellement, les réactifs 1, 2 et 3 sont mélangés.

Selon une variante avantageuse, le réactif la est constitué par de l'annexine V. Dans ce cas, le tampon de dilution du réactif 3 est un tampon calcique, par exemple constitué par un mélange Hepes/NaCl/CaCl2, car la fixation de l'annexine aux phospholipides est dépendante de la présence de calcium (15). On évitera évidemment dans ce cas d'utiliser un échantillon traité par l'EDTA.

Le réactif 1b est avantageusement composé d'un mélange d'ACM de spécificités différentes, et préférentiellement anti-CD61 et anti-CD42b.
Plus particulièrement, le réactif 1b est constitué par un mélange d'ACM P18, 4F8 (anti-CD61) et SZ2 (anti-CD42b).

On utilise de préférence le FITC comme fluorochrome 1 et la PE comme fluorochrome 2.

Pour assurer une bonne conservation, tous les réactifs du kit de l'invention peuvent contenir de l'azide de sodium à 0,09 % (0,09 g/l).

Selon un autre aspect complémentaire, l'invention a pour objet un procédé de détection et de quantification des MPP, caractérisé en ce qu'il comprend les étapes suivantes :
a) mise en présence et incubation d'un échantillon sanguin avec un monoréactif tel que défini ci-dessus, de sorte à obtenir un marquage en double couleur desdites MPP, et
b) analyse cytométrique des événements doublement marqués.

L'invention vise enfin l'utilisation d'un monoréactif tel que défini précédemment ou d'un kit de diagnostic comprenant ledit monoréactif, dans une méthode de détection et de suivi d'un état prothrombotique.

Le principe de la méthode d'analyse cytométrique utilisée dans le cadre de l'invention est rappelé ci-après :

### Analyse cytométrique :

Pour effectuer la lecture cytométrique, se rapporter au protocole d'utilisation de l'appareil fourni par le fabricant.
Avant l'analyse, homogénéiser les tubes à l'aide d'un agitateur de type Vortex.
Pour la réalisation du protocole, sont nécessaires :
- 1 cytogramme FSXSS
- 2 histogrammes FL1 LOG, un conditionné par le cytogramme FSXSS (pour les billes A de positionnement) et l'autre conditionné par le cytogramme FSXSS (pour les billes B de comptage)
- 2 cytogrammes FL1 LOG/FL2 LOG, un cytogramme conditionné sur les billes seuil (C et D) et l'autre conditionné sur les billes A + MPPs.

Construire un cytogramme FS LOG x SS LOG. Positionner un seuil discriminant pour éliminer les éventuels contaminants (bruit de fond de l'appareil). Dessiner 3 fenêtres d'analyse autour des différentes populations de billes :
- Fenêtre R1 pour les billes de positionnement de 0,8µm
- Fenêtre R2 pour les billes de comptage de 5 à 10 µm
- Fenêtre R3 pour les billes seuils de 3µm.

Créer 2 histogrammes FL1 LOG, un pour les billes de positionnement (conditionné par la fenêtre R1), l'autre pour les billes de comptage (conditionné par la fenêtre R2).

Créer 2 cytogrammes FL1 LOG/FL2 LOG, un cytogramme pour les billes seuils C et/ou D (conditionné par la fenêtre R3) et l'autre pour les MPPs (conditionné par la fenêtre R1).

Pour des conditions d'analyses optimales, ajuster le voltage du photomultiplicateur FL1 et FL2 de telle sorte que la bille haute seuil (C ou D) soit positionnée au début de la 4ème décade en FL1 ou FL2 respectivement.
Le réglage des compensations est effectué sur les billes seuils (C et D).

### Réglage de la compensation FL2

Sur le cytogramme FL1 LOG/FL2 LOG conditionné sur la fenêtre d'analyse "R3" des billes D, positionner 2 fenêtres (R4 et R5) , sur les nuages correspondants, respectivement, à la bille haute et à la bille basse. Compenser la fluorescence FL2 (FL2 -x % FL1) jusqu'à équivalence des moyennes de fluorescence (MFI) FL2 LOG des fenêtres R4 et R5.

### Réglage de la compensation FL1

Ne pas changer les réglages de fluorescence FL1 LOG, FL2 LOG (voltages des photomultiplicateurs, PMT FL1 et FL2) précédemment fixés, pour le reste du protocole.
Procéder de la même manière que précédemment décrit. Sur le cytogramme FL1 LOG/FL2 LOG conditionné sur la fenêtre d'analyse "R3" des billes C, positionner 2 fenêtres (R6 et R7) , sur les nuages correspondants, respectivement, à la bille haute et à la bille basse. Compenser la fluorescence FL1 (FL1 -x % FL2) jusqu'à équivalence des MFI FL1 LOG des fenêtres R6 et R7.

Les exemples ci-après, illustrent la présente invention.

### Exemple n°1 : Etude des billes seuils d'un monoréactif selon l'invention.

Le monoréactif illustré ci-après est basé sur un double marquage des MPP par l'annexine V-FITC et le CD 41-PE.

### 1.1 Préparation des billes seuils

Les billes seuils ont été préparées à partir de billes de polystyrène de 3µm coatées à l'aide de quantités différentes d'une IgG murine non réactive avec les éléments figurés du sang. La charge en IgG murine est choisie selon le niveau d'intensité de fluorescence recherché pour cette bille seuil. Les billes sont marquées en fluorescence par contact avec un réactif anti IgG murines correspondant soit à :
- FITC labeled sheep F(ab')2 anti-mouse IgG (H+L), human absorbed
- FITC labeled goat Fab anti-mouse IgG (H+L), human absorbed
- PE labeled sheep F(ab')2 anti-mouse IgG (H+L), human absorbed
- PE labeled goat Fab anti-mouse IgG (H+L), human absorbed

### a. Préparation des billes coatées IgG

Le lot de billes est choisi pour donner une intensité moyenne de fluorescence décalée par rapport à une population de plaquettes non marquées (ex. moyenne de fluorescence des billes 10 fois plus fortes que la moyenne de fluorescence des plaquettes).

### Matériel

- AcM Sendo-3, anti-CD146, clone F439-E10, non réactif avec les éléments figurés du sang (Leucocyte Typing VI, Kishimoto, Kikutani et al.)
- Billes L300 (Estapor)
Tampons (tableau 1):

| **TAMPONS** | **SOLUTIONS** |
|---|---|
| Adsorption | PBS |
| Saturation | PBS, BSA 4 % |
| Lavage | PBS |
| Fixation | PBS, PFA 1%, azide 0,09% |
| Stockage | PBS,BSA 0,1%; azide 0,09% |
| Dilution | PBS |

### Méthode

### Préparation des tampons

Préparer 1 litre de PBS, filtrer sur membrane de 0,22 µm avec un système de filtration de 500ml.
Préparer 1 litre de PBS, PFA 1%, Azide de sodium 0,09%
Préparer 1 litre de PBS BA, filtrer sur membrane de 0,22 µm avec un système de filtration de 500ml.
NB : Tous les tampons sont filtrés sur membrane de 0,22 µm avant utilisation.

### Préparation des réactifs de couplage

a) Dilution des IgG1
   Les différents niveaux de charge des billes sont obtenus en faisant varier la concentration en IgGl des solutions de coating.
   1. Décongeler un aliquot de Sendo3 à 5mg/ml.
   2. Préparer une solution de Sendo3 à 500 µg/ml (dilution au 1/10) comme suit:
      - déposer dans un tube CMF 100 µl de Sendo3 à 5 mg/ml
      - ajouter dans ce même tube 900µl de PBS
   3. Préparer une solution de Sendo3 à 100 µg/ml (dilution au 1/5) comme suit:
      - déposer dans un tube CMF 200 µl de Sendo3 à 500 µg/ml
      - ajouter dans ce même tube 800µl de PBS
   4. Préparer les 2 solutions de coating comme indiqué dans le tableau 2: 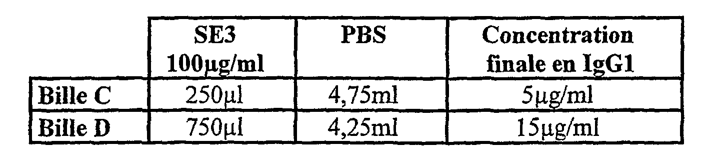
b) Préparation des microsphères avant couplage
   Protocole général de lavage des microsphères
   Les lavages et changements de tampon sont tous réalisés comme suit :
   1. Centrifuger les billes à 3000 tr / min pendant 8 min (1890g, rotor 8160), avec le frein.
   2. Eliminer le surnageant à l'aide de la trompe à vide.
   3. Avec une pipette ajouter le tampon de reprise.
   4. Bien vortexer pour remettre le culot en suspension.

### Lavage des billes avant le couplage

1. Déposer 6 ml de suspension de billes à 10 % (vérifié à environ 4,106 microsphères/µl)
2. Centrifuger, éliminer le surnageant.
3. Remettre le culot en suspension avec 7,5 ml de PBS.
4. Centrifuger, éliminer le surnageant.
5. Remettre le culot en suspension avec 6 ml de PBS.

### Protocole de couplage

a. Vortexer le tube contenant les microsphères lavées.
b. Vortexer le tube contenant la solution de coating.
c. Maintenir le tube contenant la solution de coating en agitation sur le vortex. Ajouter rapidement 1ml de microsphères lavées avec une Gilson de lml, en une seule fois, verticalement au coeur de la solution.
d. Boucher le tube.
e. Positionner le tube sur l'agitateur rotatif à +2-8°C.
f. Répéter les étapes b à e avec les autres solutions de coatings.
g. Incuber 1 nuit en agitation.
h. Préparer le tampon de saturation (2g de BSA A7030 qsp 50ml de PBS), filtrer 0,22µm avec un filtre.
i. Filtrer 100 ml de tampon de fixation sur membrane 0,22 µm avec un filtre seringue.
j. Centrifuger, Eliminer les surnageants (Voir Protocole général de lavage des microsphères).
k. Reprendre les culots avec 5 ml de tampon de saturation, Vortexer.
l. Centrifuger, Eliminer les surnageants.
m. Reprendre les culots avec 10 ml de PBS, Vortexer.
n. Centrifuger, Eliminer les surnageants.
o. Reprendre les culots avec 10 ml de tampon de fixation, Vortexer.
p. Incuber 10 min. à TA en agitation.
q. Centrifuger, Eliminer les surnageants.
r. Reprendre les culots avec 10 ml de PBS-BA, Vortexer.
s. Centrifuger, Eliminer les surnageants.
t. Reprendre les culots avec 10 ml de PBS-BA, Vortexer.
u. Centrifuger, Eliminer les surnageants.
v. Reprendre les culots avec 7,5 ml de PBS-BA, Vortexer

Les suspensions individuelles (solution à environ 500000 microsphères /µl) sont prêtes et sont stockées à +2-8°C.

### b. Préparation des billes seuils fluorescentes à l'aide de F(ab')2 conjugués au FITC ou à la PE

### Matériel

- F(ab')2 d'immunoglobulines de mouton anti-souris couplées au FITC (Silenus , réf. DDAF)
- F(ab')2 d'immunoglobulines de mouton anti-souris couplées à la PE (Silenus, réf. DDAPE)

### Méthode

1. Pipeter 2,5 ml de bille L300 (Estapor) coatées en Sendo-3 (300.000 microsphères/µl)
   Sendo-3 à 15 µg/ml pour la bille FITC
   Sendo-3 à 5 µg/ml pour la bille PE
2. Centrifugation à 3000t/minute pendant 8 minutes. Elimination du surnageant
3. Reprise dans 500 µl de tampon PBS BA 1X
4. Ajouter 12,5 ml de DDAF ou DDAPE au 1/100 en PBS BA 1X à la préparation de bille. Homogénéiser.
5. Incuber 1 heure à température ambiante sous agitation rotative
6. Effectuer 2 lavages comme suit :
   Ajouter 25 ml de PBS BA 1X
   Centrifugation à 3000t/minute pendant 8 minutes.
   Elimination du surnageant.
7. Ajouter 2,5 ml d'une solution de gamma globuline de souris (Jackson, #015-000-002) à 100 µg/ml sur le culot de billes, afin de neutraliser les sites anti-IgG de souris libres des réactifs DDAF et DDAPE
8. Incuber 1 heure à température ambiante sous agitation rotative
9. Effectuer 1 lavage comme décrit ci-dessus
10. Reprendre le culot de billes dans 2,5 ml de PBS BA 1X.

### c. Préparation des billes seuils à l'aide de Fab conjugués au FITC ou à la PE

### Matériel

- Fab d'immunoglobulines de chèvre anti-souris couplées au FITC (Protos, réf. #341)
- Fab d'immunoglobulines de chèvre anti-souris couplées à la PE (Protos, réf. #441)

### Méthode

Comme précédemment, sans les étapes 7, 8 et 9.

### 1.2 Analyse cytométrique

Les 2 types de billes seuils FITC et PE préparées ont été testées seules en tampon PBS BA ou en présence de l'ensemble des autres réactifs constituant le monoréactif (AcMx couplés FITC ou PE et billes A de 0,85 µm de positionnement). Les paramètres de scatters (scatter axial noté ici FS, scatter perpendiculaire noté ici SS) sont analysés en échelle logarithmique, avec un discriminateur réglé sur SS.

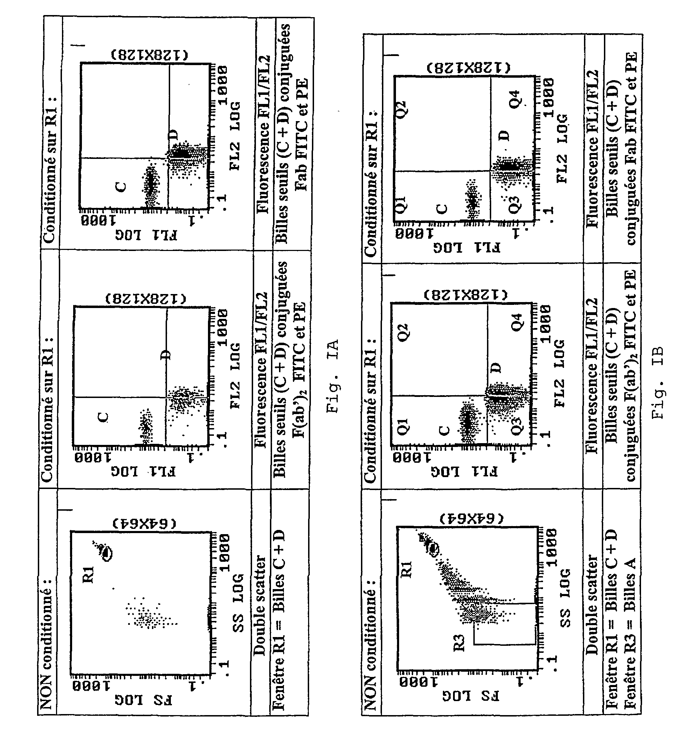

Les résultats sont reportés sur la figure 1, dans laquelle :
- la figure 1a) représente l'analyse des billes seuils en tampon PBS BA.
- la figure 1b) représente l'analyse des billes seuils avec les billes A dans le monoréactif.
Dans ces figures, la fenêtre R1 correspond aux billes seuils FITC et PE de 3 µm, la fenêtre R3 à la fenêtre d'analyse des MPP en double scatter, les billes D aux billes seuils FITC et les billes C aux billes seuils PE.

### 1.3 Conclusion

Il est possible d'utiliser le mélange de billes 3 µm marquées en FITC et PE comme décrit pour positionner une fenêtre (notée ici Q2) d'analyse d'éléments doublement marqués. Dans l'exemple, le seuil sur FL2 log (seuil vertical) a été choisi au niveau de la valeur moyenne des billes D, le seuil sur FL1 log (seuil horizontal) a été choisi égal à 1/3.5 la valeur moyenne des billes C.

La fonctionnalité d'une association extemporanée des différentes catégories de billes (billes seuils FITC et PE de 3 µm permettant de positionner une zone de mesure Q2 et billes FITC de 0,85µm permettant de positionner la fenêtre R3 d'analyse des MPP) nécessaires à la réalisation du test en un monoréactif est démontrée.

### Exemple n°2 : Test du monoréactif selon l'invention.

### 2.1 Matériel

- **CD61 :** P18-FITC (purifié et marqué selon les procédures standards connues de l'homme de métier) (réactif filtré 0,1µm)
   P18 (McGregor JL, Thromb.Haemost. 1987, 58, 507)
- **CD41a :** P2-PE (Réf. IM1416, Immunotech)
   P2 (Réf. IM0145, Immunotech)
- **Annexin V FITC** (Réf. BMS306FI/a, Bender Medsystems)
- **Billes de positionnement (A) de 0,85µm** (Cat. VFP 08525 Avidin coated Yellow, Spherotec)
- **Billes seuils (C et D) conjuguées F(ab')2 FITC et PE** précédemment décrites
- **Tampon CaCl2 1X** (Hepes 10mM, NaCl 140mM, CaC12 2.5mM)
- **Tampon CaCl2 EDTA** (Hepes 10mM, NaCl 140mM, CaCl2 2.5mM, EDTA 6,2mM pH7,4)

### 2.2 Protocole

1. Pipeter 30 µl de Sang total prélevé sur Citrate de sodium ou EDTA et dilué au 1/10 en PBS BA 1X
2. Pipeter 10 µl AcMx non couplés ou de tampon (PBS BA 1X ou CaCl2 ou CaCl2 EDTA)
3. Incuber 10 minutes à température ambiante.
4. Ajouter 10 µl AcM couplé au FITC ou Annexin V couplé au FITC.
5. Pipeter 10 µl AcM couplé à la PE.
6. Pipeter 5 µl de billes seuil FITC (200000 microsphères/□l)
7. Pipeter 5 µl de billes seuil PE (200000 microsphères/□l)
8. Pipeter 5 µl de billes Spherotec de 0,85µm diluées au 1/20 en PBS BA 1X
9. Incuber 20 minutes à température ambiante.
10. Ajouter 1 ml de tampon (PBS BA 1X ou CaCl2 ou CaCl2 EDTA)
11. Analyse cytométrique : temps d'analyse de 2 minutes, vitesse de flux lente.

### Remarque :

1. Dans le cadre de ces essais de monoréactif, les billes seuils jouent le rôle de billes de comptage des MPP.
2. Dans le cas des inhibitions de fixation des AcMx couplés aux fluorochromes, une pré-incubation de 10 minutes à température ambiante de l'échantillon sanguin avec les AcMx non couplés est réalisée.

### 2.3 Conditions Opératoires

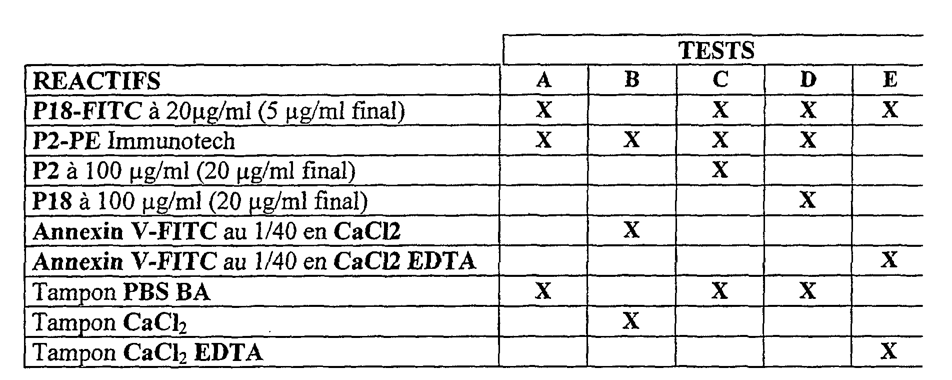

**Le test A** permet de numérer les MPP et les plaquettes de taille inférieure ou égale à 0,85 µm et correspondant à des événements CD61 +/ CD41a +.

**Le test B** permet de numérer les MPP et/ou les plaquettes activées de taille inférieure ou égale à 0,85 µm correspondant à des évènements Annexin V +/ CD41a +. Seul ce test permet de mettre en évidence les éléments d'origine plaquettaire issus du sang, de taille inférieure ou égale à 0,85 µm et présentant un phénotype activé (de par la présence au niveau de la membrane cellulaire externe des plaquettes et des MPP de résidus Phosphatidylserine révélés par l'annexin V).
**Les tests C, D, et E** permettent d'établir la spécificité des réactifs CD41a, CD61, et AnnexinV respectivement.

### 2.4 Test du monoréactif en sang total

### a) Principe de positionnement de la fenêtre R3 d'analyse des MPP en double scatter en sang total

L'exemple ci-dessous illustre le principe d'analyse des MPP en double scatter en sang total. L'échantillon sanguin correspond au test A (CD61-FITC/CD41-PE).

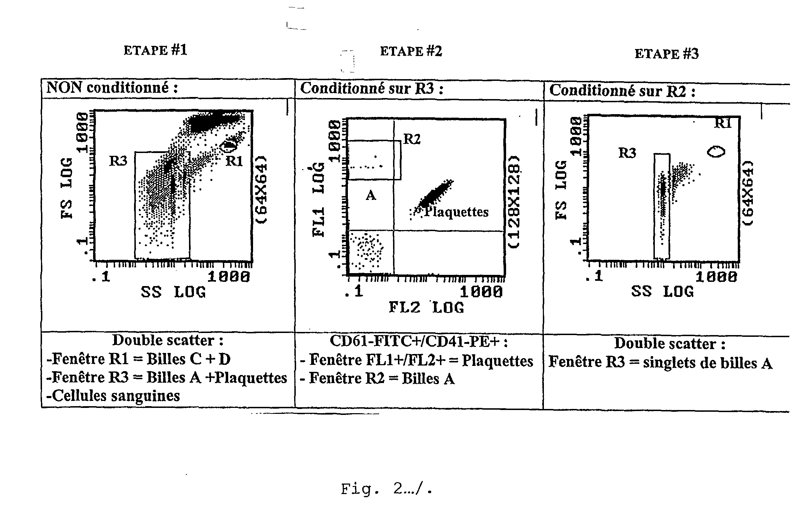

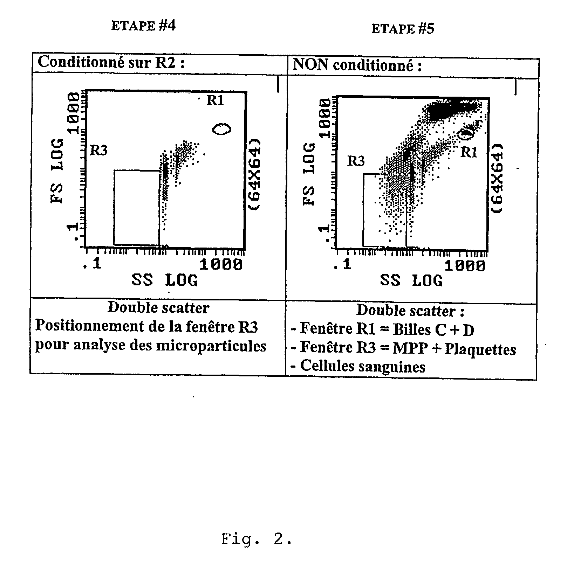

Les différentes étapes de l'analyse cytométrique sont reportées sur la figure 2.

La fenêtre **R3** d'analyse des MPP est conditionnée à l'aide des singlets de billes de positionnement de 0,85µm, fluorescentes FITC dans la masse (Billes A). Une large fenêtre R3 en double scatter est tout d'abord conditionnée autour de ces billes (ETAPE #1). Un cytogramme FL1-LOG/FL2-LOG conditionné sur R3 permet de repérer en FL1 ces billes de positionnement par une fenêtre R2 (ETAPE #2). Une fenêtre R3 est alors redéfinie en double scatter et une mesure de la valeur moyenne du paramètre FS Log est faite sur cette population de singlet de billes A (ETAPE #3).

La fenêtre R3 d'analyse des microparticules plaquettaires peut alors être positionnée en double scatter selon les critères suivants (ETAPE #4) :
- Limite haute FS-LOG : égale à la valeur moyenne du paramètre FS LOG pour la population de singlet de billes A, mesurée dans l'étape précédente.
- Limite basse FS-LOG : ne pas prendre le premier canal du paramètre FS LOG.
- Limite haute SS-LOG : contre le nuage de singlet de billes A
- Limite basse SS-LOG : discriminateur sur le paramètre SS LOG réglé au minimum

Enfin, l'analyse en double scatter d'échantillons (sang total + monoréactif) avec les différentes fenêtres d'analyses cytométriques peut être faite (ETAPE #5).

### b. Analyses d'échantillons

Le test de la fonctionnalité du monoréactif et de la spécificité de ses composants a été réalisé sur 19 sangs totaux : 11 sangs prélevés sur **EDTA** et 7 sangs prélevés **sur citrate de sodium.**
Les tests A, B, C, D, et E ont été effectués selon le protocole décrit ci-dessus, à partir de sang dilué au 1/10.

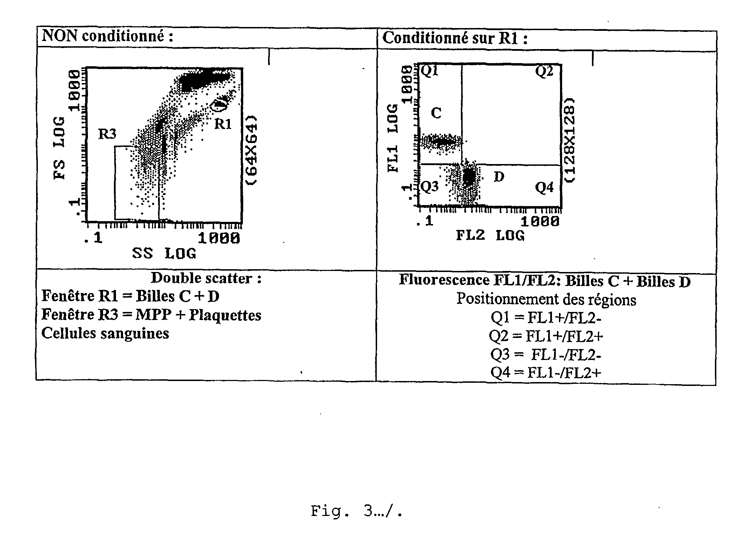

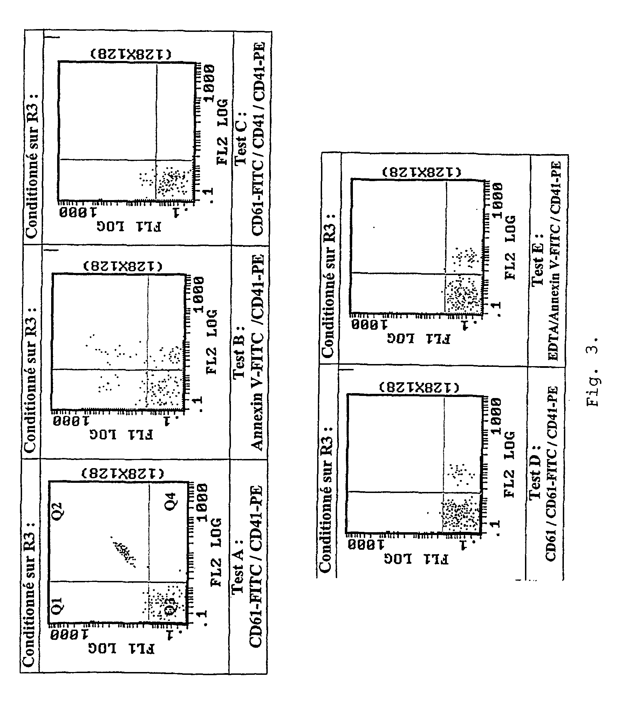

**Résultats cytométriques** sont reportés sur la figure 3.

**Résultats quantitatifs** sont exprimés en nombre d'événements par µl de sang total.

L'analyse est effectuée à partir des éléments numérés dans la fenêtre Q2.
- **Anticoagulant EDTA :**

| **Echantillon** | **Test A** | **Test B** |
|---|---|---|
| 1 | 6113 | 1094 |
| 2 | 6030 | 1540 |
| 3 | 6776 | 1628 |
| 4 | 9060 | 1115 |
| 5 | 7428 | 974 |
| 6 | 5158 | 1822 |
| 7 | 3131 | 1636 |
| 8 | 4401 | 1189 |
| 9 | 5777 | 666 |
| 10 | 12981 | 756 |
| 11 | 7105 | 504 |
| **Moyenne** | **6724** | **1175** |
| **Ecart Type** | **2603** | **436** |

- **Anticoagulant Citrate Na :**

| **Echantillon** | **Test A** | **Test B** |
|---|---|---|
| 12 | 7002 | 716 |
| 5 | 5610 | 879 |
| 13 | 3205 | 400 |
| 9 | 8095 | 1190 |
| 10 | 10680 | 781 |
| 14 | 5344 | 960 |
| 15 | 13620 | 896 |
| **Moyenne** | **7651** | **832** |
| **Ecart Type** | **3528** | **243** |

- **Tests d'inhibition** (anticoagulant EDTA) :

| | | | **Pourcentage d'inhibition** | | |
|---|---|---|---|---|---|
| **Echantillon** | **Test A** | **Test B** | **Test C** | **Test D** | **Test E** |
| **2** | 6030 | 1540 | **99,32%** | **99,25%** | **97,01%** |
| **3** | 6776 | 1628 | **98,86%** | **98,27%** | **94,35%** |
| **4** | 9060 | 1115 | **98,45%** | **98,65%** | **92,56%** |
| **6** | 5158 | 1822 | **96,76%** | **93,68%** | **98,30%** |
| **7** | 3131 | 1636 | **95,02%** | **93,42%** | **93,46%** |
| **8** | 4401 | 1189 | **96,68%** | **94,75%** | **95,63%** |

### REFERENCES

**1.** Assembly of the Platelet Prothrombinase Complex Is Linked to Vesiculation of the Platelet Plasma Membrane. Studies in Scott syndrome : an isolated defect in platelet procoagulant activity". P.J. Sims, T. Wiedmer, C.T. Esmon, H.J. Weiss, and S. J. Shattil. J. Biol. Chem., vol. 264, no. 29,17049-17057,1989.
**2.** Microparticle generation during in vitro platelet activation by anti-CD9 murine monoclonal antibodies. S. Nomura, H. Nagata, M. Suzuki, K. Kondo, S. Ohga, T. Kawakatsu, H. Kido, T. Fukuroi, K. Yamaguchi, K. Twata, M. Yanabu, T. Soga, T. Kokawa, and K. Yasunaga. Thrombosis Research, 62, 429-439, 1991.
**3.** Platelet Microparticles Bind, Activate and Aggregate Neutrophils In Vitro. W. Jy, W.-W. Mao, L.L. Horstman, J. Tao, Y.S. Ahn. Blood Cells, Molecules and Diseases. 21, 217-231, 1995.
**4.** A new lupus anticoagulant neutralization test based on platelet-derived vesicles. J. Arnout, E. Huybrechts, M. Vanrusselt, and J. Vermylen. Bristish Journal of Haematology. 80, 341-346, 1992.
**5.** Platelet procoagulant activity and microvesicle formation. Its putative role in hemostasis and thrombosis. R.F.A. Zwaal, P. Comfurius and E. M. Bevers. Biochimica et Biophysica Acta. 1180, 1-8, 1992.
**6.** Demonstration of Platelet-Derived Microvesicles in Blood from Patients with Activated Coagulation and Fibrinolysis Using a Filtration Technique and Western Blotting. P.A. Holme, N.O. Solum, F. Brosstad, M. Roger. Thrombosis and Haemostasis.72 (5), 666-71, 1994.
**7.** WO 96/03655 (8 février 1996) - Inventeurs : J.M. Freyssinet, B. Antoni, F. Donie, H. Lill.
**8.** Direct Detection of Activated Platelets and Platelet-Derived Microparticles in Humans. C. S. Abrams, N. Ellison, A.Z. Budzyndki, and S.J. Shattil. Blood, vol. 75, no. 1, 128-138, 1990.
**9.** Annexin V as a Probe of Aminophopholipid Exposure and Platelet Membrane Vesiculation : A flow Cytometry Study Showing a Role for Free Sulfhydryl Groups. J. Dachary-Prigent, J.-M. Freyssinet, J.-M. Pasquet, J.-C. Carron, and A. T. Nurden. Blood, vol. 81, no. 10, 2554-2565, 1993.
**10.** Platelet-derived Microvesicles in Thrombotic Thrombocytopenic Purpura and Hemolytic Uremic Syndrome. M. Galli, A. Grassi, T. Barbui. Thrombosis and Haemostasis. 75 (3), 427-31, 1996.
**11.** A New Flow Cytometry Method of Platelet-derived Microvesicle Quantification in Plasma. V. Combes, F. Dignat-George, M. Mutin, J. Sampol. Thrombosis and Haemostasis. 77 1, 212-24, 1997.
**12.** WO 96/15449 (23 mai 1996) - Inventeurs A.D. Michelson, M.R. Barnard.
**13.** Terminology and Nomenclature for Standardization in Quantitative Fluorescence Cytometry. L.O. Henderson, G.E. Marti, A. Gaigalas, W.H. Hannon, R.F. Vogt Jr., Cytometry, 33:97-105, 1998.
**14.** Antiphospholipid antibody binding to bilayer-coated glass microspheres. A.R.Obringer, N.S. Rote, A. Walter , J. Immunol. Methods, 185 1, 81-93, 1995.
**15.** Ca2+ concentration during binding determines the manner in which annexin V binds to membrane. P.J. Trotter, M.A. Orchard, J.H. Walker Biochem J., 308, 591-8, 1995.

## Revendications

1. Monoréactif pour la détection et la quantification des microparticules plaquettaires (MPP) dans un échantillon sanguin par cytométrie de flux, comprenant :
a) un réactif 1 pour un double marquage des MPP comprenant :
- soit de l'Annexine V ou tout autre marqueur spécifique des phospholipides membranaires couplé à un fluorochrome 1,
- soit une population 1 d'ACM dirigés contre des structures membranaires plaquettaires marqués avec un fluorochrome 1, (réactif 1a), et,
une population 2 d'anticorps monoclonaux (ACM) dirigés contre des structures membranaires plaquettaires marqués avec un fluorochrome 2 (réactif 1b), et,
b) un réactif 2 constitué par un mélange de microsphères comprenant :
- une population de microsphères A marquée par l'un des deux fluorochromes 1 ou 2 ou autre fluorochrome de spectre similaire à l'un des deux, servant à définir la région d'analyse des MPP en terme de taille (paramètres de diffusion lumineuse) et permettant leur différenciation (billes de positionnement),
- une population de microsphères B non marquée ou marquée par l'un des deux fluorochromes 1 ou 2 ou autre fluorochrome de spectre similaire à l'un des deux, dont la concentration est connue, servant d'étalon interne au comptage des microparticules (billes de comptage),
- une population de microsphères C marquée uniquement par le fluorochrome 1, servant à définir sur le paramètre de fluorescence du fluorochrome 1 le seuil d'intensité minimum qui délimite la région d'analyse des microparticules sur lesquelles sont fixés les ACM 1 marqués au fluorochrome 1 ou l'Annexine V ou autre marqueur spécifique des phospholipides membranaires marqués au fluorochrome 1 (billes seuils),
- et une population de microsphères D, de diamètre identique à C, et marquée uniquement par le fluorochrome 2, servant à définir sur le paramètre de fluorescence du fluorochrome 2 le seuil d'intensité minimum qui délimite la région d'analyse des microparticules sur lesquelles sont fixés les ACM 2 (billes seuils).

2. Monoréactif selon la revendication 1 **caractérisé en ce que** chaque population C et D de billes seuils est composée de deux sous-populations de billes de deux niveaux.

3. Monoréactif selon la revendication 1 **caractérisé en ce que** les billes de comptage ont un diamètre supérieur ou égal à 5 µm.

4. Monoréactif selon la revendication 1 **caractérisé en ce que** les billes de comptage ont un diamètre compris entre 5 et 10 µm.

5. Monoréactif selon la revendication 1 **caractérisé en ce que** les billes de positionnement ont un diamètre compris entre 1 et 0,5 µm.

6. Monoréactif selon la revendication 1, **caractérisé en ce que** les billes seuils ont un diamètre de 3 µm.

7. Monoréactif selon la revendication 1, **caractérisé en ce que** les anticorps monoclonaux (ACM) des populations 1 et 2 sont dirigés contre des structures membranaires plaquettaires choisies parmi les spécificités suivantes : CD61, CD41, CD42a, CD42b, CD42c, CD49b, CD29, CD62P, CD63, protéine S et prothrombine.

8. Monoréactif selon la revendication 1, **caractérisé en ce que** les populations 1 et 2 d'ACM sont constituées par un seul ACM, par plusieurs ACM de même spécificité mais dirigés contre des épitopes différents, ou par plusieurs ACM de spécificité plaquettaire différente.

9. Monoréactif selon la revendication 1, **caractérisé en ce que** le réactif 1 du monoréactif de l'invention est constitué d'annexine V ou autre marqueur spécifique des phospholipides membranaires marqué par un fluorochrome 1 (réactif 1a), et d'une population 2 d'ACM marqués par un fluorochrome 2 (réactif 1b).

10. Monoréactif selon la revendication 1, **caractérisé en ce que** le réactif 1 est constitué par de l'annexine V marquée par un fluorochrome 1 et d'une population 2 d'ACM marqués par un fluorochrome 2.

11. Monoréactif selon la revendication 10, **caractérisé en ce que** la population 2 d'ACM est constituée par plusieurs ACM de spécificités différentes.

12. Monoréactif selon la revendication 10, **caractérisé en ce que** la population 2 d'ACM est constituée par des ACM anti-CD61 et des ACM anti-CD42b.

13. Monoréactif selon la revendication 10, **caractérisé en ce que** la population 2 est constituée par les ACM P18 et 4F8 (anti-CD61) et SZ2 (anti-CD42b).

14. Monoréactif selon l'une des revendications 1 à 13, **caractérisé en ce que** le fluorochrome 1 est le FITC et le fluorochrome 2 la PE.

15. Monoréactif selon l'une des revendications 1 à 14, **caractérisé en ce que** l'échantillon est du sang total.

16. Kit de diagnostic pour la détection et la quantification des MPP, **caractérisé en ce qu'**il comprend un monoréactif selon l'une quelconque des revendications 1 à 15.

17. Kit selon la revendicationl6, **caractérisé en ce qu'**il comprend en outre un tampon de dilution.

18. Kit selon la revendication 16, **caractérisé en ce qu'**il comprend :
- **un réactif 1a** constitué par une population 1 d'ACM spécifiques de structures membranaires plaquettaires, ou par de l'annexine V ou tout autre marqueur spécifique des phospholipides membranaires, marqués avec un fluorochrome 1,
- **un réactif 1b** constitué par une population 2 d'ACM spécifiques de structures membranaires plaquettaires, marqués avec un fluorochrome 2,
- **un réactif 2** constitué par :
• une population de billes de positionnement de la fenêtre d'analyse des MPP marquée par le fluorochrome 1 ou 2 ou autre fluorochrome de spectre similaire à l'un des deux,
• une population de billes de comptage non marquée ou marquée par le fluorochrome 1 ou 2 ou autre fluorochrome de spectre similaire à l'un des deux,
• une population de billes seuils, préférentiellement constituée par deux sous-populations de billes de deux niveaux, marquées par le fluorochrome 1,
• une population de billes seuils, préférentiellement constituée par deux sous-populations de billes de deux niveaux marquées par le fluorochrome 2.
- **un réactif 3** constitué par un tampon de dilution.

19. Kit selon l'une des revendications 16 à 18, **caractérisé en ce que** le réactif la est constitué par de l'annexine V et le réactif 1b par un mélange d'ACM de spécificités différentes.

20. Kit selon l'une des revendications 16 à 18, **caractérisé en ce que** le réactif 1b est constitué par un mélange d'ACM anti-CD61 et anti-CD42b.

21. Procédé de détection et de quantification des MPP, **caractérisé en ce qu'**il comprend les étapes suivantes :
a) mise en présence et incubation d'un échantillon sanguin avec un monoréactif selon l'une quelconque des revendications 1 à 15 de sorte à obtenir un marquage en double couleur desdites MPP, et,
b) analyse cytométrique des événements doublement marqués.

22. Utilisation d'un monoréactif selon l'une quelconque des revendications 1 à 15 ou d'un kit de diagnostic selon l'une quelconque des revendications 16 à 20 dans une méthode de détection et de suivi d'un état prothrombotique.

## Patentansprüche

1. Monoreagenz für den Nachweis und die Quantifizierung von Blutplättchen-Mikroteilchen (BMT) in einer Blutprobe mittels Durchflusszytometrie, umfassend:
a) ein Reagenz für eine doppelte Markierung der BMT, umfassend:
- entweder Annexin V oder jeden anderen Marker, der für Membran-Phospholipide spezifisch ist, gekuppelt an ein Fluorochrom 1,
- oder eine Population 1 von MAK, die gegen Blutplättchen-Membranstrukturen gerichtet sind, markiert mit einem Fluorochrom 1 (Reagenz 1a), und
eine Population 2 von monoklonalen Antikörpern (MAK), die gegen Blutplättchen-Membranstrukturen gerichtet sind, markiert mit einem Fluorochrom 2 (Reagenz 1b), und
b) ein Reagenz 2, das aus einer Mischung von Mikrokügelchen zusammengesetzt ist, welche umfassen:
- eine Population von Mikrokügelchen A, die mit einem der beiden Fluorochrome 1 oder 2 oder einem anderen Fluorochrom mit einem Spektrum, das einem dieser beiden ähnlich ist, markiert ist und dazu dient, den Analysebereich der BMT bezüglich der Größe (Lichtdiffusionsparameter) zu definieren, und deren Differenzierung ermöglicht (Positionierungskügelchen),
- eine Population von Mikrokügelchen B, die nicht markiert oder mit einem der beiden Fluorochrome 1 oder 2 oder einem anderen Fluorochrom mit einem Spektrum, das einem der beiden ähnlich ist, markiert ist, deren Konzentration bekannt ist und die als innerer Standard zur Zählung der Mikroteilchen dient (Zählungskügelchen),
- eine Population von Mikrokügelchen C, die allein mit dem Fluorochrom 1 markiert ist und dazu dient, bei dem Fluoreszenzparameter des Fluorochroms 1 die minimale Intensitätsschwelle zu definieren, welche den Analysebereich der Mikroteilchen beschränkt, an denen die mit dem Fluorochrom 1 markierten MAK 1 oder Annexin V oder ein anderer, für Membran-Phospholipide spezifischer Marker, markiert mit dem Fluorochrom 1, befestigt sind (Schwellenkügelchen),
- eine Population von Mikrokügelchen D mit einem mit C identischen Durchmesser und allein mit dem Fluorochrom 2 markiert, die dazu dient, bei dem Fluoreszenzparameter des Fluorochroms 2 die minimale Intensitätsschwelle zu definieren, die den Analysebereich der Mikroteilchen beschränkt, an denen die MAK 2 befestigt sind (Schwellenkügelchen).

2. Monoreagenz nach Anspruch 1, **dadurch gekennzeichnet, dass** jede Population C und D von Schwellenkügelchen aus zwei Unterpopulationen von Kügelchen mit zwei Niveaus zusammengesetzt ist.

3. Monoreagenz nach Anspruch 1, **dadurch gekennzeichnet, dass** die Zählungskügelchen einen Durchmesser von größer oder gleich 5 µm aufweisen.

4. Monoreagenz nach Anspruch 1, **dadurch gekennzeichnet, dass** die Zählungskügelchen einen Durchmesser zwischen 5 und 10 µm einschließlich aufweisen.

5. Monoreagenz nach Anspruch 1, **dadurch gekennzeichnet, dass** die Positionierungskügelchen einen Durchmesser zwischen 1 und 0,5 µm einschließlich aufweisen.

6. Monoreagenz nach Anspruch 1, **dadurch gekennzeichnet, dass** die Schwellenkügelchen einen Durchmesser von 3 µm aufweisen.

7. Monoreagenz nach Anspruch 1, **dadurch gekennzeichnet, dass** die monoklonalen Antikörper (MAK) der Populationen 1 und 2 gegen Blutplättchen-Membranstrukturen gerichtet sind, die aus den folgenden Spezifitäten ausgewählt sind: CD61, CD41, CD42a, CD42b, CD42c, CD49b, CD29, CD62P, CD63, Protein S und Prothrombin.

8. Monoreagenz nach Anspruch 1, **dadurch gekennzeichnet, dass** die MAK-Populationen 1 und 2 aus einem einzigen MAK, aus mehreren MAK der gleichen Spezifität, aber gegen unterschiedliche Epitope gerichtet, oder aus mehreren MAK mit unterschiedlicher Blutplättchen-Spezifität zusammengesetzt sind.

9. Monoreagenz nach Anspruch 1, **dadurch gekennzeichnet, dass** das Reagenz 1 des Monoreagenz der Erfindung aus Annexin V oder einem anderen, für Membran-Phospholipide spezifischen, mit einem Fluorochrom 1 markierten Marker (Reagenz 1a) und einer Population 2 von mit einem Fluorochrom 2 markierten MAK (Reagenz 1 b) zusammengesetzt ist.

10. Monoreagenz nach Anspruch 1, **dadurch gekennzeichnet, dass** das Reagenz 1 aus mit einem Fluorochrom 1 markiertem Annexin V und einer Population 2 von mit einem Fluorochrom 2 markierten MAK zusammengesetzt ist.

11. Monoreagenz nach Anspruch 10, **dadurch gekennzeichnet, dass** die MAK-Population 2 aus mehreren MAK mit unterschiedlichen Spezifitäten zusammengesetzt ist.

12. Monoreagenz nach Anspruch 10, **dadurch gekennzeichnet, dass** die MAK-Population 2 aus Anti-CD61-MAK und Anti-CD42b-MAK zusammengesetzt ist.

13. Monoreagenz nach Anspruch 10, **dadurch gekennzeichnet, dass** die Population 2 aus MAK P18 und 4F8 (Anti-CD61) und SZ2 (Anti-CD42b) zusammengesetzt ist.

14. Monoreagenz nach einem der Ansprüche 1 bis 13, **dadurch gekennzeichnet, dass** das Fluorochrom 1 FITC ist und das Fluorochrom 2 PE ist.

15. Monoreagenz nach einem der Ansprüche 1 bis 14, **dadurch gekennzeichnet, dass** die Probe Vollblut ist.

16. Diagnostisches Kit zum Nachweis und zur Quantifizierung von BMT, **dadurch gekennzeichnet, dass** es ein Monoreagenz nach irgendeinem der Ansprüche 1 bis 15 umfasst.

17. Kit nach Anspruch 16, **dadurch gekennzeichnet, dass** es darüber hinaus einen Verdünnungspuffer umfasst.

18. Kit nach Anspruch 16, **dadurch gekennzeichnet, dass** es umfasst:
- **ein Reagenz 1a,** das aus einer Population 1 von für Blutplättchen-Membranstrukturen spezifischen MAK oder aus Annexin V oder aus einem anderen, für Membran-Phospholipide spezifischen Marker, die mit einem Fluorochrom 1 markiert sind, zusammengesetzt ist,
- **ein Reagenz 1b,** das aus einer Population 2 von für Blutplättchen-Membranstrukturen spezifischen MAK, die mit einem Fluorochrom 2 markiert sind, zusammengesetzt ist,
- **ein Reagenz 2,** das zusammengesetzt ist aus:
• einer Population von Positionierungskügelchen mit einem Fenster zur Analyse von BMT, die mit dem Fluorochrom 1 oder 2 oder einem anderen Fluorochrom mit einem Spektrum, das einem der beiden ähnlich ist, markiert ist,
• einer Population von Zählungskügelchen, die nicht markiert oder mit dem Fluorochrom 1 oder 2 oder einem anderen Fluorochrom mit einem Spektrum, das einem der beiden ähnlich ist, markiert ist,
• einer Population von Schwellenkügelchen, die vorzugsweise aus zwei Unterpopulationen von Kügelchen mit zwei Niveaus zusammengesetzt ist, welche mit dem Fluorochrom 1 markiert sind,
• einer Population von Schwellenkügelchen, die vorzugsweise aus zwei Unterpopulationen von Kügelchen mit zwei Niveaus zusammengesetzt ist, welche mit dem Fluorochrom 2 markiert sind,
- **ein Reagenz 3**, das aus einem Verdünnungspuffer zusammengesetzt ist.

19. Kit nach einem der Ansprüche 16 bis 18, **dadurch gekennzeichnet, dass** das Reagenz 1a aus Annexin V und das Reagenz 1b aus einer Mischung von MAK mit unterschiedlichen Spezifitäten zusammengesetzt ist.

20. Kit nach einem der Ansprüche 16 bis 18, **dadurch gekennzeichnet, dass** das Reagenz 1b aus einer Mischung von Anti-CD61- und Anti-CD42b-MAK zusammengesetzt ist.

21. Verfahren zum Nachweis und zur Quantifizierung von BMT, **dadurch gekennzeichnet, dass** es die folgenden Schritte umfasst:
a) Vereinigen und Inkubieren einer Blutprobe mit einem Monoreagenz nach irgendeinem der Ansprüche 1 bis 15 auf solche Weise, dass man eine Markierung der BMT mit doppelter Farbe erhält, und
b) zytometrische Analyse der doppelt markierten Ereignisse.

22. Verwendung eines Monoreagenz nach irgendeinem der Ansprüche 1 bis 15 oder eines Diagnosekits nach irgendeinem der Ansprüche 16 bis 20 in einem Verfahren zum Nachweis und zur Verfolgung eines prothrombotischen Zustands.

## Claims

1. Monoreagent for detecting and quantifying platelet-derived microparticles (PMPs) in a blood sample by flow cytometry, comprising:
a) a reagent 1 for double labelling the PMPs, comprising:
- either annexin V, or any other marker specific for membrane phospholipids, coupled to a fluorochrome 1,
- or a population 1 of MABs, directed against platelet membrane structures, labelled with a fluorochrome 1, (reagent 1a), and
- a population 2 of monoclonal antibodies (MABs) , directed against platelet membrane structures, labelled with a fluorochrome 2 (reagent 1b), and,
b) a reagent 2 consisting of a mixture of microspheres comprising:
- a population of microspheres A labelled with one of the two fluorochromes 1 and 2, or another fluorochrome with a spectrum similar to one of the two, used to define the region of analysis of the PMPs in terms of size (light scattering parameters) and allowing differentiation thereof (setting beads),
- a population of microspheres B, unlabelled or labelled with one of the two fluorochromes 1 and 2, or another fluorochrome with a spectrum similar to one of the two, the concentration of which is known, used as internal standard for counting the microparticles (counting beads),
- a population of microspheres C labelled only with fluorochrome 1, used to define, with respect to the fluorochrome 1 fluorescence parameter, the minimum intensity threshold which delimits the region of analysis of the microparticles to which the MABs 1 labelled with fluorochrome 1 or the annexin V, or any other marker specific for membrane phospholipids. labelled with fluorochrome 1, are bound (threshold beads),
- and a population of microspheres D, of diameter identical to C, and labelled only with fluorochrome 2, used to define, with respect to the fluorochrome 2 fluorescence parameter, the minimum intensity threshold which delimits the region of analysis of the microparticles to which the MABs 2 are bound (threshold beads).

2. Monoreagent according to Claim 1, **characterized in that** each population C and D of threshold beads is composed of two subpopulations of beads with two levels.

3. Monoreagent according to Claim 1, **characterized in that** the counting beads are greater than or equal to 5 µm in diameter.

4. Monoreagent according to Claim 1, **characterized in that** the counting beads are between 5 and 10 µm in diameter.

5. Monoreagent according to Claim 1, **characterized in that** the setting beads are between 1 and 0.5 µm in diameter.

6. Monoreagent according to Claim 1, **characterized in that** the threshold beads are 3 µm in diameter.

7. Monoreagent according to Claim 1, **characterized in that** the monoclonal antibodies (MABs) of the populations 1 and 2 are directed against platelet membrane structures chosen from the following specificities: CD61, CD41, CD42a, CD42b, CD42c, CD49b, CD29, CD62P, CD63, protein S and prothrombin.

8. Monoreagent according to Claim 1, **characterized in that** the populations 1 and 2 of MABs consist of a single MAB, of several MABs with the same specificity but directed against different epitopes, or of several MABs with different platelet specificities.

9. Monoreagent according to Claim 1, **characterized in that** reagent 1 of the monoreagent of the invention consists of annexin V, or another marker specific for membrane phospholipids, labelled with a fluorochrome 1 (reagent 1a), and of a population 2 of MABs labelled with a fluorochrome 2 (reagent 1b).

10. Monoreagent according to Claim 1, **characterized in that** reagent 1 consists of annexin V labelled with a fluorochrome 1 and of a population 2 of MABs labelled with a fluorochrome 2.

11. Monoreagent according to Claim 10, **characterized in that** the population 2 of MABs consists of several MABs with different specificities.

12. Monoreagent according to Claim 10, **characterized in that** the population 2 of MABs consists of anti-CD61 MABs and anti-CD42b MABs.

13. Monoreagent according to Claim 10, **characterized in that** the population 2 consists of the MABs P18 and 4F8 (anti-CD61) and SZ2 (anti-CD42b).

14. Monoreagent according to one of Claims 1 to 13, **characterized in that** fluorochrome 1 is FITC and fluorochrome 2 is PE.

15. Monoreagent according to one of Claims 1 to 14, **characterized in that** the sample is whole blood.

16. Diagnostic kit for detecting and quantifying PMPs, **characterized in that** it comprises a monoreagent according to any one of Claims 1 to 15.

17. Kit according to Claim 16, **characterized in that** it also comprises a dilution buffer.

18. Kit according to Claim 16, **characterized in that** it comprises:
- **a reagent 1a** consisting of a population 1 of MABs specific for platelet membrane structures, or annexin V or any other marker specific for membrane phospholipids, labelled with a fluorochrome 1,
- **a reagent 1b** consisting of a population 2 of MABs specific for platelet membrane structures, labelled with a fluorochrome 2,
- **a reagent 2** consisting of:
• a population of beads for setting the analysis window for the PMPs, labelled with fluorochrome 1 or 2, or another fluorochrome with a spectrum similar to one of the two,
• a population of counting beads, unlabelled or labelled with fluorochrome 1 or 2, or another fluorochrome with a spectrum similar to one of the two,
• a population of threshold beads, preferentially consisting of two subpopulations of beads with two levels, labelled with fluorochrome 1,
• a population of threshold beads, preferentially consisting of two subpopulations of beads with two levels, labelled with fluorochrome 2,
- **a reagent 3** consisting of a dilution buffer.

19. Kit according to .one of Claims 16 to 18, **characterized in that** reagent 1a consists of annexin V and reagent 1b consists of a mixture of MABs with different specificities.

20. Kit according to one of Claims 16 to 18, **characterized in that** reagent 1b consists of a mixture of anti-CD61 and anti-CD42b MABs.

21. Method for detecting and quantifying PMPs, **characterized in that** it comprises the following steps:
a) bringing a blood sample into contact with a monoreagent according to any one of Claims 1 to 15 and incubating them, so as to obtain double colour labelling of said PMPs, and
b) cytometrically analysing the double labelled events.

22. Use of a monoreagent according to any one of Claims 1 to 15, or of a diagnostic kit according to any one of Claims 16 to 20, in a method for detecting and monitoring a prothrombotic condition.
